(19) **Europäisches Patentamt / European Patent Office / Office européen des brevets**

(11) **EP 4 722 690 A1**

(12) **EUROPEAN PATENT APPLICATION**
published in accordance with Art. 153(4) EPC

(43) Date of publication:
08.04.2026 Bulletin 2026/15

(21) Application number: 24815513.7

(22) Date of filing: 29.05.2024

(51) International Patent Classification (IPC):
$G01N\ 7/00^{(2006.01)}$  $C10M\ 169/02^{(2006.01)}$
$C10N\ 40/00^{(2006.01)}$  $C10N\ 50/10^{(2006.01)}$

(52) Cooperative Patent Classification (CPC):
C10M 169/02; G01N 7/00; C10N 2040/00;
C10N 2050/10

(86) International application number:
PCT/JP2024/019681

(87) International publication number:
WO 2024/248032 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.05.2023 JP 2023088048

(71) Applicant: NSK LTD.
Tokyo 141-8560 (JP)

(72) Inventors:
• MAEDA Masayuki
Fujisawa-shi, Kanagawa 251-8501 (JP)
• NAKAGAWA Kazunori
Fujisawa-shi, Kanagawa 251-8501 (JP)
• MARUYAMA Taisuke
Fujisawa-shi, Kanagawa 251-8501 (JP)

(74) Representative: Grünecker Patent- und
Rechtsanwälte
PartG mbB
Leopoldstraße 4
80802 München (DE)

(54) **GREASE EVAPORATION AMOUNT PREDICTION METHOD, PREDICTION DEVICE, AND PROGRAM**

(57) This grease evaporation amount prediction method comprises: a calculation step for calculating a change in the amount of grease by using the lesser between a first substance amount and a second substance amount, the first substance amount being obtained by a first formula based on a vapor pressure drop caused by a base oil and a thickener included in the grease, and the second substance amount being obtained by a second formula based on a capillary force according to the thickener; and an output step for outputting the calculated change in amount as the amount of evaporation of the grease.

FIG. 3

EP 4 722 690 A1

**Description**

TECHNICAL FIELD

The present invention relates to a prediction method, a prediction device, and a program for predicting an evaporation amount of grease.

BACKGROUND ART

In the related art, in a mechanical device such as a bearing device or a sliding device, a lubricant is used for the purpose of reducing friction in the operation thereof. An example of the lubricant is grease. The components of the grease may evaporate over time. As the grease evaporates, the amount and viscosity of the grease change, which may affect the operation of the mechanical device.

For example, Patent Literature 1 discloses, as a configuration of grease, a configuration of a grease composition excellent in low torque performance at low temperatures and oxidation stability.

CITATION LIST

PATENT LITERATURE

Patent Literature 1: JP5319995B

SUMMARY OF INVENTION

TECHNICAL PROBLEM

In order to stabilize the operation of a mechanical device and predict the life of the mechanical device, it is required to predict a change amount due to evaporation of the grease used. In addition, in order to newly design grease for a mechanical device, it is also required to easily predict the change amount due to evaporation of the grease when considering conditions related to evaporation. However, the change amount due to evaporation of the grease and the prediction thereof are not considered in Patent Literature 1.

In view of the above problems, an object of the present invention to is provide a method capable of easily predicting a change amount of grease due to evaporation.

SOLUTION TO PROBLEM

In order to solve the above problems, the present invention has the following configuration, that is, a prediction method for predicting an evaporation amount of grease, the prediction method including:

a calculation step of calculating a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output step of outputting the calculated change amount as an evaporation amount of the grease.

Further, another aspect of the present invention has the following configuration, that is, a prediction device for predicting an evaporation amount of grease, the prediction device including:

a calculation unit configured to calculate a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output unit configured to output the calculated change amount as an evaporation amount of the grease.

Further, another aspect of the present invention has the following configuration, that is, a program for causing a computer to execute:

a calculation step of calculating a change amount of grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output step of outputting the calculated change amount as an evaporation amount of the grease.

ADVANTAGEOUS EFFECTS OF INVENTION

According to the present invention, the evaporation amount of grease can be easily predicted.

BRIEF DESCRIPTION OF DRAWINGS

FIG. 1 is a conceptual diagram for illustrating modeling of grease according to an embodiment of the present invention.
FIG. 2 is a graph illustrating an example of a test result related to evaporation of grease.
FIG. 3 is a graph illustrating an example of a test result related to evaporation of grease.
FIG. 4 is a graph illustrating another example of a test result related to evaporation of grease.
FIG. 5 is a graph illustrating fitting when deriving parameters according to an embodiment of the present invention.
FIG. 6A is a graph illustrating an influence of a parameter on evaporation according to an embodiment of the present invention.
FIG. 6B is a graph illustrating an influence of a parameter on evaporation according to the embodiment of the present invention.
FIG. 6C is a graph illustrating an influence of a parameter on evaporation according to an embodiment of the present invention.
FIG. 6D is a graph illustrating an influence of a parameter on evaporation according to an embodiment of the present invention.
FIG. 6E is a graph illustrating an influence of a parameter on evaporation according to an embodiment of the present invention.
FIG. 7 is a block diagram illustrating a configuration example of a device capable of executing a method for predicting an evaporation amount of grease according to an embodiment of the present invention.

DESCRIPTION OF EMBODIMENTS

Hereinafter, embodiments of the present invention will be described with reference to the drawings. The embodiments described below are embodiments for explaining the present invention, and are not intended to be interpreted to limit the present invention, and all the configurations described in the embodiments are not necessarily essential configurations for solving the problems of the present invention. In the drawings, the same components are denoted by the same reference signs, thereby showing a correspondence relation therebetween.

<First Embodiment>

Hereinafter, a first embodiment of the present invention will be described. Grease as a lubricant described in the present embodiment can be used for a rolling bearing, a rotation device, a sliding device, and the like, but is not limited thereto. The method according to the present embodiment can be used in an environment in which grease is used or designed, and the use of the method is not particularly limited.

Hereinafter, a description will be given using a plurality of equations. Parameters in the equations may partially overlap in expression. In this case, content of the parameter is indicated in association with the corresponding equation. The unit and granularity of variables are also examples and can be appropriately replaced.

[Modeling of Grease]

The grease mainly contains a base oil and a thickener. Although other components can also be contained in the grease, these components are considered to be extremely little with respect to content ratios of the base oil and the thickener, and a description thereof will be omitted here.

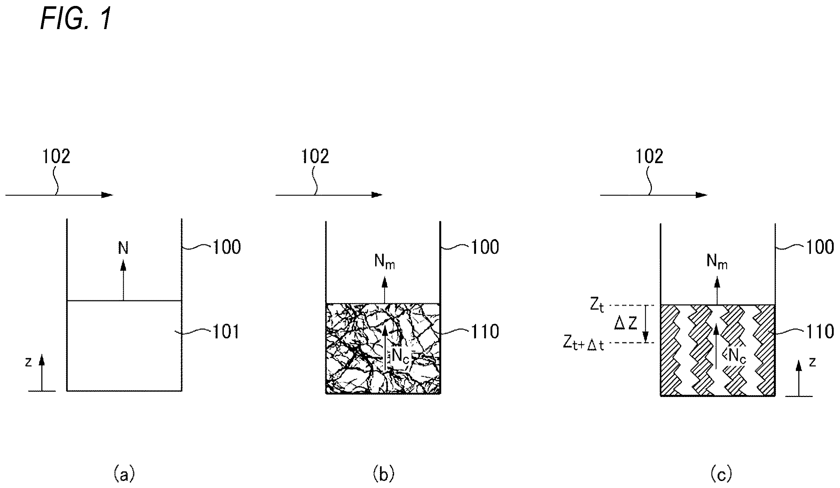

FIG. 1 is a schematic diagram illustrating modeling of grease. In FIG. 1, (a) illustrates an example in which a base oil 101, which is a main component of grease, is contained in any container 100. The container 100 has a configuration in a cylindrical shape and is open at an upper portion. An arrow 102 indicates a gas flow around the container 100. In this case, the base oil 101 may vaporize from its liquid surface and flow out to the outside. When the base oil 101 vaporizes and flows out, a liquid surface height z changes (decreases).

In FIG. 1, (b) illustrates an example in which grease 110 is contained in the container 100. As described above, the grease 110 contains a base oil and a thickener, and has a different behavior of evaporation from the base oil as illustrated in (a) of FIG. 1 due to the configuration. More specifically, the behavior of evaporation of the grease 110 can be defined by a relationship between evaporation of the base oil based on a vapor pressure depression at the liquid surface and movement of the base oil due to a capillary force based on a structure of the thickener in the grease 110.

In general, the vapor pressure of a solution (here, grease) in which a nonvolatile solute (here, thickener) is dissolved is lower than that of a pure solvent (here, base oil as illustrated in (a) of FIG. 1). Such a phenomenon is called a vapor pressure depression, which is also considered in the present embodiment.

In FIG. 1, (c) illustrates a model of the grease as a curved flow path model in consideration of the structure of the thickener with respect to (b) of FIG. 1. When the liquid surface height at a certain time point t is defined as $z_t$, the liquid surface height after elapse of a time t (= $\Delta t$) from the time point t is represented by $z_{t+\Delta t}$. A change amount of the liquid surface height in this case is $\Delta z$ (= $z_t$ - $z_{t+\Delta t}$).

[Test Related to Evaporation]

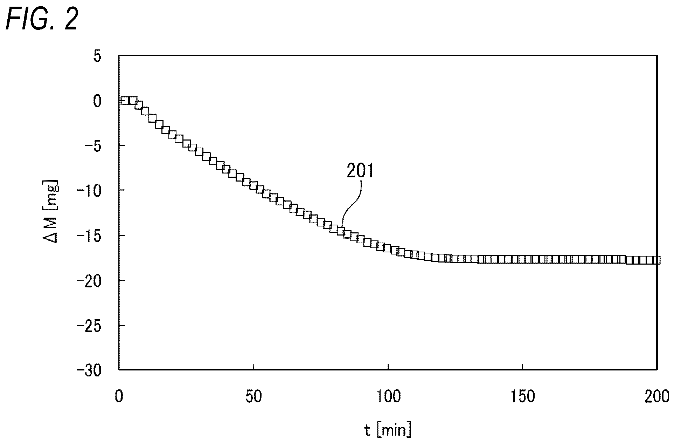

FIG. 2 is a graph illustrating a test result related to evaporation of grease under the following test conditions. In FIG. 2, a horizontal axis represents time t [min], and a vertical axis represents a change amount $\Delta M$ [mg] of the grease. A plot 201 indicates an actual measurement value of the change amount of the grease.

(Test Conditions)

Grease type: PAO (polyalphaolefin)-urea grease
Consistency: 250
Thickener ratio: 16.8 [%]
Initial amount: 21.49 [mg]
Ambient temperature: 180 [°C]
Atmosphere gas: $N_2$
Gas flow rate: 100 [ml/min]

As a result of the above test, the change amount $\Delta M$, that is, an evaporation amount was stable at 17.88 mg. The remaining amount of the grease at this time is 16.79% (= (21.49-17.88)/21.49), which is substantially equal to the ratio of the thickener contained in the grease. Therefore, when considering the evaporation of the grease, the behavior of the evaporation of the base oil in the grease may be considered. In the present embodiment, focusing on the evaporation of the base oil in the grease, the parameters related to the evaporation are set and the calculation formula is constructed.

In the model illustrated in (c) of FIG. 1, when the base oil in the grease 110 is vaporized and the amount of its gas moved is defined as the amount of the grease 110 moved, that is, the amount of the grease 110 (base oil) evaporated, the evaporation amount of the grease 110 can be defined by the following Equation (1).

[Math. 1]

$$0 - NA = \frac{\rho A}{M}\frac{dz}{dt} \quad \cdot \cdot \cdot \quad (1)$$

N: molar mass by which liquid moves per unit time and per unit area [mol/s·$m^2$]
A: cross-sectional area of particle layer [$m^2$]
$\rho$: density of liquid (here, base oil) [g/$m^3$]
M: molecular weight of liquid (here, base oil) [g/mol]

In Equation (1), "0" on the left side means that there is no inflow of gas into the grease 110, and "NA" on the left side indicates outflow of gas to the outside of the grease 110. That is, a case is assumed where the grease 110 evaporates and flows out of the container 100. The right side is the amount of the grease 110 moved, which corresponds to $\Delta z$ illustrated in (c) of FIG. 1.

Then, based on Equation (1), the liquid surface height after the elapse of t ($\Delta t$) from the certain time point t can be defined by the following Equation (2).

[Math. 2]

$$z_{t+\Delta t} = z_t + \Delta z = z_t - \frac{M}{\rho} N \Delta t \quad \cdots \quad (2)$$

$z_t$: liquid surface height at time point t [m]
$z_{t+\Delta t}$: liquid surface height after elapse of time t from time point t [m]
$\Delta z$: change amount of z [m]
M: molecular weight [g/mol]
$\rho$: density of liquid (here, base oil) [g/m$^3$]
N: molar mass by which liquid moves per unit time and per unit area [mol/s·m$^2$]
$\Delta t$: elapsed time [s]

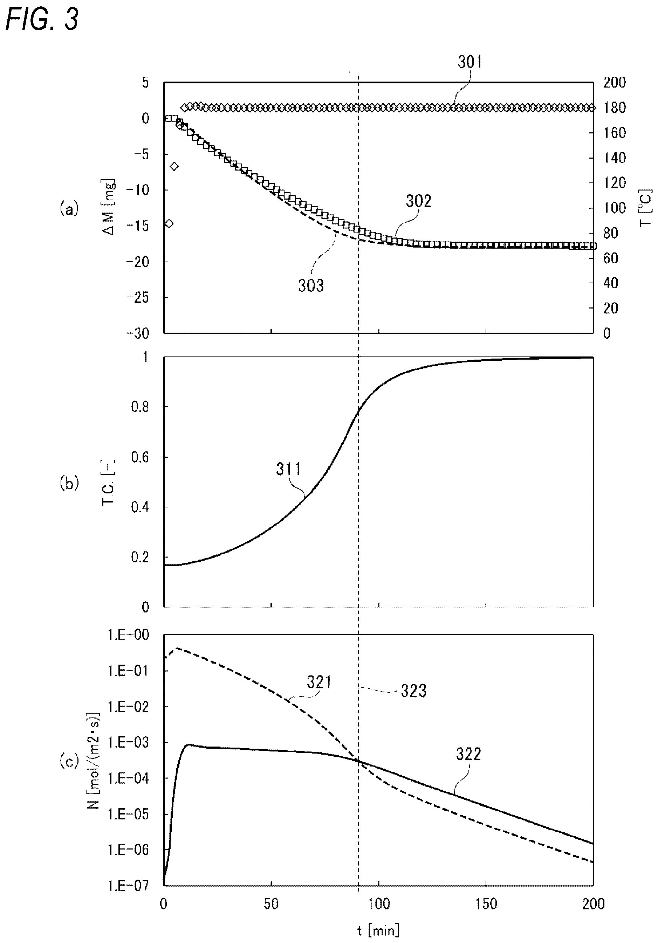

Here, a parameter N included in Equations (1) and (2) will be described. As described above, the evaporation of the grease can be defined by a relationship between a molar mass (hereinafter referred to as "$N_m$") in the evaporation of the base oil based on the vapor pressure depression at the liquid surface and a molar mass (hereinafter referred to as "$N_c$") in the movement of the base oil by the capillary force based on the structure of the thickener in the grease. These relationships will be described with reference to FIGS. 3 to 6E.

(Test 1)

FIG. 3 is a graph illustrating test results related to evaporation of grease under the following test conditions. The grease used in Test 1 is referred to as "grease A" for convenience.

Base oil: PAO2
Thickener: Urea (C18-MDI)
Thickener ratio: 16.8 [%]
Kinematic viscosity: 5.54 (at 40°C), 1.9 (at 100°C) [mm$^2$/s]
Consistency: 250
Temperature: 180 [°C]
Atmosphere gas: $N_2$
Gas flow rate: 100 [ml/min]
Container height: 5 [mm]

In (a) of FIG. 3 to (c) of FIG. 3, horizontal axes represent time t [min], and are corresponding to each other. A vertical axis in (a) of FIG. 3 represents the change amount $\Delta M$ [mg]. A vertical axis in (b) of FIG. 3 represents a concentration [-]. A vertical axis in (c) of FIG. 3 represents the parameter N [mol/m$^2$·s] related to evaporation.
In (a) of FIG. 3, a plot 301 indicates an actual measurement value of the temperature, and a plot 302 indicates an actual measurement value of the evaporation amount of the grease A. A solid line 311 in (b) of FIG. 3 indicates a calculated value of a thickener concentration in the grease A. That is, a ratio of the thickener to the entire grease A is shown. A solid line 321 in (c) of FIG. 3 indicates the molar mass ($N_c$) in the movement of the base oil due to the capillary force caused by the structure of the thickener in the grease A. A solid line 322 in (c) of FIG. 3 indicates the molar mass ($N_m$) in the evaporation of the base oil in consideration of the vapor pressure depression. As indicated by the solid line 321 and the solid line 322, at a certain time point after a lapse of time from a reference time (t = 0), the molar mass ($N_c$) in the movement of the base oil due to the capillary force and the molar mass ($N_m$) in the evaporation of the base oil in consideration of the vapor pressure depression have a switch in magnitude. Initially, the molar mass ($N_c$) in the movement of the base oil due to the capillary force is larger, but after a certain time elapses, the molar mass ($N_m$) in the evaporation of the base oil in consideration of the vapor pressure depression is larger. The timing at which the magnitudes thereof switch is indicated by a broken line 323.

(Test 2)

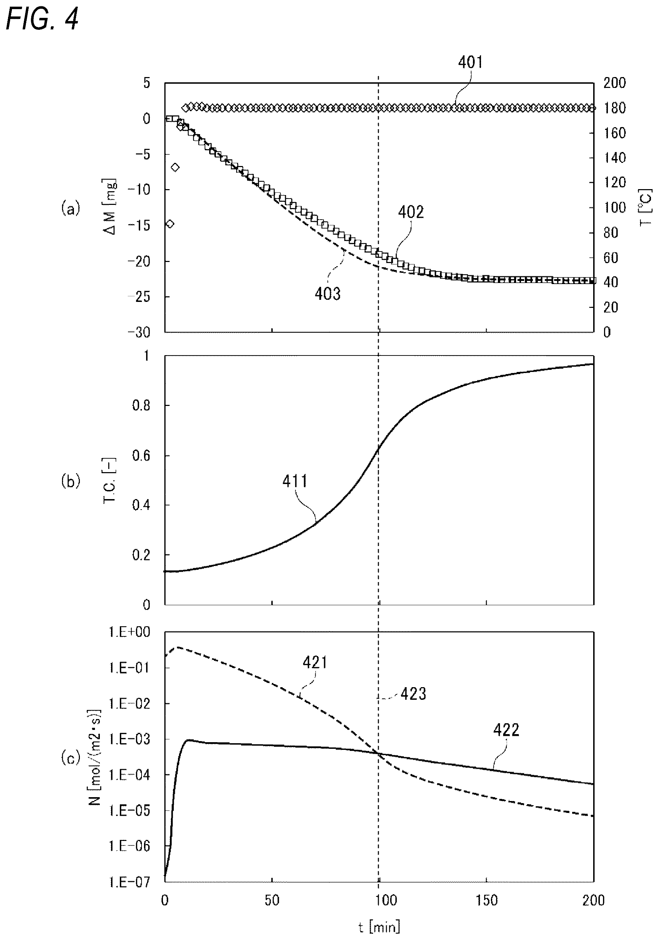

FIG. 4 is a graph illustrating test results related to evaporation of grease under the following test conditions. The grease used in Test 2 is referred to as "grease B" for convenience.

Base oil: PAO2
Thickener: Urea (C18-MDI)
Thickener ratio: 13.3 [%]
Kinematic viscosity: 5.54 (at 40°C), 1.9 (at 100°C) [mm$^2$/s]
Consistency: 350

Temperature: 180 [°C]
Atmosphere gas: $N_2$
Gas flow rate: 100 [ml/min]
Container height: 5 [mm]

The configuration of each axis in (a) of FIG. 4 to (c) of FIG. 4 is the same as that in FIG. 3. In (a) of FIG. 4, a plot 401 indicates an actual measurement value of the temperature, and a plot 402 indicates an actual measurement value of an evaporation amount of the grease B. A solid line 411 in (b) of FIG. 4 indicates a calculated value of a thickener concentration in the grease B. That is, a ratio of the thickener to the entire grease is shown. A solid line 421 in (c) of FIG. 4 indicates the molar mass ($N_c$) of the movement of the base oil due to the capillary force caused by a structure of the thickener in the grease B. A solid line 422 in (c) of FIG. 4 indicates the molar mass ($N_m$) in the evaporation of the base oil in consideration of the vapor pressure depression. Similarly to the case in FIG. 3, as indicated by the solid line 421 and the solid line 422, at a certain time point, the molar mass ($N_c$) in the movement of the base oil due to the capillary force and the molar mass ($N_m$) in the evaporation of the base oil in consideration of the vapor pressure depression have a switch in magnitude. Initially, the molar mass ($N_c$) in the movement of the base oil due to the capillary force is larger, but after a certain time elapses, the molar mass ($N_m$) in the evaporation of the base oil in consideration of the vapor pressure depression is larger. The timing at which the magnitudes thereof switch is indicated by a broken line 423.

In the present embodiment, a calculation formula for calculating a predicted value corresponding to the actual measurement value indicated by the plot 302 in (a) of FIG. 3 or the plot 402 in (a) of FIG. 4 is defined.

Regarding N in the above Equation (2), the molar mass ($N_m$) in the evaporation of the oil in consideration of the vapor pressure depression can be defined by the following Equations (3) to (6). Note that Equations (3) to (6) are based on Raoult's law and Fick's law. Since these are publicly known, a detailed description thereof will be omitted here. For details, for example, see "James R. Welty, Gregory L. Rorrer, David G. Foster, A. N. Bhaskarwar, "Fundamentals of Momentum, Heat and Mass Transfer Sixth Edition', wiley, 2013." Here, the base oil as a liquid is also referred to as a liquid A, and the base oil as a gas is also referred to as a gas A. The gas around the container is also referred to as a gas B. Therefore, in the case of the example in (b) of FIG. 1, the liquid A corresponds to the base oil in the grease 110, and the gas A corresponds to the vaporized base oil in the container 100. The flow of the gas B is indicated by the arrow 102.

[Math. 3]

$$N_m = \frac{D_{AB} P'_{vap}}{y_{B,lm} RTZ} \quad \cdots (3)$$

$$P'_{vap} = \frac{n_{oil}}{n_{oil} + n_{thick}} P_{vap} \quad \cdots (4)$$

$$D_{AB} = (1.858 \times 10^{-4}) \frac{\left(\frac{1}{M_A} + \frac{1}{M_B}\right)^{\frac{1}{2}} T^{\frac{3}{2}}}{P \sigma_{AB}^2 \Omega_D} \quad \cdots (5)$$

$$y_{B,lm} = \frac{y_{B2} - y_{B1}}{\ln\left(\frac{y_{B2}}{y_{B1}}\right)} \quad \cdots (6)$$

P: absolute pressure [Pa]
$P_{vap}$: vapor pressure of liquid A (base oil) [Pa]
$n_{oil}$: molar mass of liquid A (base oil) [mol]
$n_{thiek}$: molar mass of thickener [mol]
$D_{AB}$: two-component (gas A and gas B) diffusion coefficient [m²/s]
$y_B$, lm: values that change due to influence of convection in atmosphere gas (gas B) [-]
R: gas constant [J/K·mol]
T: temperature [K]
Z: distance from top surface of container to liquid surface (= $z_2$ - $z_1$) [m]
$z_1$: liquid surface height [m]
$z_2$: container height (> $z_1$) [m]

$y_{B1}$: molar fraction of gas B at position of liquid surface height $z_1$ [-]
$y_{B2}$: molar fraction of gas B at container height $z_2$ [-]
$M_A$: molecular weight of gas A [g/mol]
$M_B$: molecular weight of gas B [g/mol]
$\sigma_{AB}$: collision distance (= $(\sigma_A + \sigma_B)/2$) [nm]
$\sigma_A$: molecular diameter of gas A [nm]
$\sigma_B$: molecular diameter of gas B [nm]
$\Omega_D$: collision integral [-]

Regarding N in the above Equation (2), the molar mass $N_c$ in the movement of the base oil due to the capillary force can be defined by the following Equations (7) and (8).

[Math. 4]

$$N_c = \frac{1}{2}\sqrt{\frac{\delta\varepsilon^2}{k'k^3(1-\varepsilon)^{2+m}}\frac{\rho T \cos\theta}{M\mu z}} \quad \cdot\cdot\cdot\,(7)$$

$$k = 2k'\left(\frac{z_a}{z}\right) \quad \cdot\cdot\cdot\,(8)$$

$\delta$: fitting parameter [m²]
$\varepsilon$: particle layer void ratio [-]
k: fitting parameter [-]
k': fitting parameter [-]
m: fitting parameter [-]
$\rho$: density of liquid [g/m³]
T: temperature [K]
$\theta$: contact angle of liquid to particle [rad.]
M: molecular weight [g/mol]
$\mu$: viscosity of liquid [Pa·s]
z: length of particle layer [m]
$z_a$: average length of curved flow path [m]

The constant k' is derived based on the evaporation test of the base oil as illustrated in FIG. 2. In the present embodiment, the smaller one of $N_m$ and $N_c$ is used as N in Equation (2) as shown in Equation (9) below.
[Math. 5]

$$N = Min(N_m, N_c) \quad \cdot\cdot\cdot\,(9)$$

[Fitting Parameter]

Here, the fitting parameters $\delta$, k, and m included in Equations (7) and (8) will be described. In the present embodiment, these fitting parameters are obtained by performing a centrifugal oil separation test on the assumption of the capillary force and performing fitting on an actual measurement value. In the centrifugal oil separation test, grease is sealed in a container provided with a filter at an opening, and the container is rotated such that the filter faces outward around a rotation axis to apply a centrifugal force. At this time, oil separation occurs in the grease due to a difference between the centrifugal force and the capillary force of the grease. The test is performed until the grease does not separate, that is, until the outward centrifugal force and the inward capillary force coincide with each other. For details on centrifugal oil separation tests, see, for example, "Soma Minoru et al., 'Measurement of grease permeability using centrifugal oil separation tests,' Tribology Conference 2018 Autumn Proceedings" and "Soma Minoru et al., 'Measurement of grease permeability using centrifugal oil separation tests (Second report)' Tribology Conference 2019 Spring Proceedings."
Based on the oil separation test, an oil separation degree of the grease can be defined by the following equations. First, a pressure gradient due to the centrifugal force acting on the grease is defined by the following Equation (10).
[Math. 6]

$$\frac{\Delta p_e}{L} = \frac{\omega^2 \rho}{L} \int_{R-L}^{R} R dR \quad \cdots (10)$$

$\Delta P_c/L$: pressure gradient due to centrifugal force acting on grease [Pa/m]
L: thickness of grease in centrifugal direction [m]
$\omega$: rotational angular velocity [rad/s]
$\rho$: density of solution [kg/m$^3$]
R: rotation radius [m]

The pressure gradient due to the capillary force acting on the grease is defined by the following Equations (11) and (12).

[Math. 7]

$$\frac{\Delta p_c}{Lk} = \frac{2T \cos \theta}{r_e Lk} \quad \cdots (11)$$

$$r_e = \sqrt{\frac{8k^2 K_p}{\varepsilon}} \quad \cdots (12)$$

$\Delta P_c/Lk$: pressure gradient due to capillary force acting on grease [Pa/m]
L: thickness of grease in centrifugal direction [m]
k: fitting parameter [-]
$K_p$: permeability [m$^2$]
T: surface tension [kg/s$^2$]
$\theta$: contact angle of liquid to particle [rad.]
$\varepsilon$: particle layer void ratio [-]

The pressure gradient acting on the grease is obtained as a difference between Equation (10) and Equation (11) using Equation (13).
[Math. 8]

$$\frac{\Delta p_e}{L} - \frac{\Delta p_c}{Lk} = \frac{\Delta p}{L'} \quad \cdots (13)$$

Further, based on Darcy's law, an oil separation degree S can be defined by the following Equations (14) to (16).

[Math. 9]

$$\frac{dV}{dt} = A \frac{K_p}{\mu_0} \frac{\Delta p}{L'} \quad \cdots (14)$$

$$K_p = \delta \frac{\varepsilon^3}{(1-\varepsilon)^{2+m}} \quad \cdots (15)$$

$$S = \frac{\rho \int_0^t \frac{dV}{dt} dt}{M_{g0}} \quad \cdots (16)$$

V: oil separation volume [m$^3$]

t: time [s]

A: cross-sectional area [m$^2$]

$K_p$: permeability [m$^2$]

$\Delta P/L'$: pressure gradient acting on grease [Pa/m]

$\mu_0$: base oil viscosity [Pa·s]

$\rho$: density of liquid [g/m$^3$]

$M_{g0}$: initial amount of grease [g]

$\delta$: fitting parameter [m$^2$]

S: oil separation degree [-]

The fitting parameters $\delta$, k, and m are derived by fitting the actual measurement values of the oil separation test to the above equations, and are applied to Equation (7).

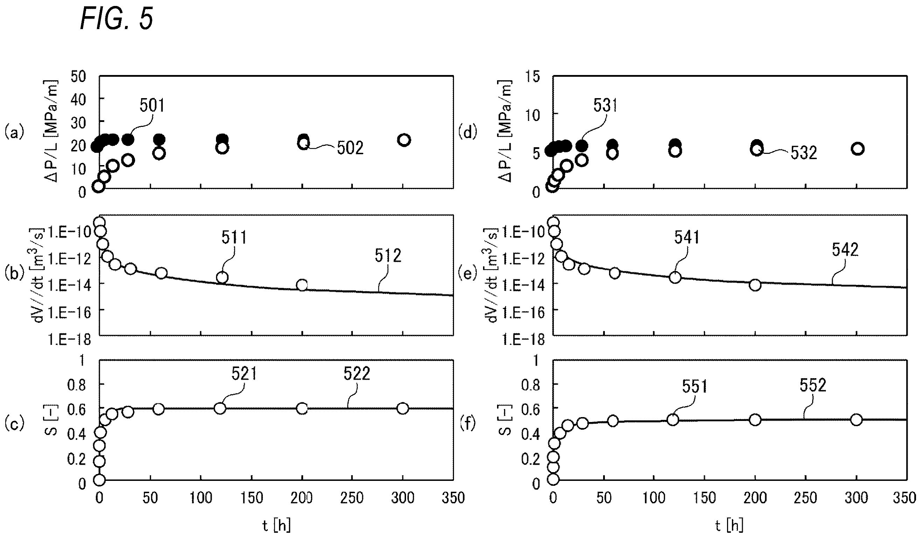

FIG. 5 illustrates an example of measurement results based on the centrifugal oil separation test as described above. In (a) of FIG. 5 to (c) of FIG. 5, measurement results of the grease A obtained at a temperature of 40°C and a rotation speed of 8000 rpm are shown. In (d) of FIG. 5 to (f) of FIG. 5, measurement results of the grease A obtained at a temperature of 40°C and a rotation speed of 4000 rpm are shown.

In (a) of FIG. 5 to (c) of FIG. 5, horizontal axes represent time [h], and are corresponding to each other. In (a) of FIG. 5, a vertical axis represents $\Delta P/L$ [MPa/m]. In (b) of FIG. 5, a vertical axis represents dV/dt [m$^3$/s]. In (c) of FIG. 5, a vertical axis represents the oil separation degree S [-].

A plot 501 indicates a pressure gradient due to centrifugal force, and a plot 502 indicates a pressure gradient due to capillary force. A plot 511 indicates an actual measurement value, and a solid line 512 indicates a value fitted thereto. Similarly, a plot 521 indicates an actual measurement value, and a solid line 522 indicates a value fitted thereto.

In (d) of FIG. 5 to (f) of FIG. 5, horizontal axes represent time [h], and are corresponding to each other. In (d) of FIG. 5, a vertical axis represents $\Delta P/L$ [MPa/m]. In (e) of FIG. 5, a vertical axis represents dV/dt [m3/s]. In (f) of FIG. 5, a vertical axis represents the oil separation degree S [-].

A plot 531 indicates a pressure gradient due to centrifugal force, and a plot 532 indicates a pressure gradient due to capillary force. A plot 541 indicates an actual measurement value, and a solid line 542 indicates a value fitted thereto. Similarly, a plot 551 indicates an actual measurement value, and a solid line 552 indicates a value fitted thereto.

From the above measurement results, as an example, the fitting parameters $\delta$, k, and m for the grease A are obtained as follows.

$$\delta = 1.56 \times 10^{-22}$$

k = 15.5

m = 7.69

When the evaporation amount of the grease A is calculated using the fitting parameters, a result indicated by a broken line 303 in (a) of FIG. 3 is obtained. This provides a calculation result with higher accuracy than the actual measurement value indicated by the plot 302.

When the fitting parameters $\delta$, k, and m for the grease B are derived by the same method, the following values are obtained as an example.

$$\delta = 3.88 \times 10^{-23}$$

k = 18.9

m = 7.64

When the evaporation amount of the grease B is calculated using the fitting parameters, a result indicated by a broken line 403 in (a) of FIG. 4 is obtained. This provides a calculation result with higher accuracy than the actual measurement value indicated by the plot 402.

[Influence of Parameters]

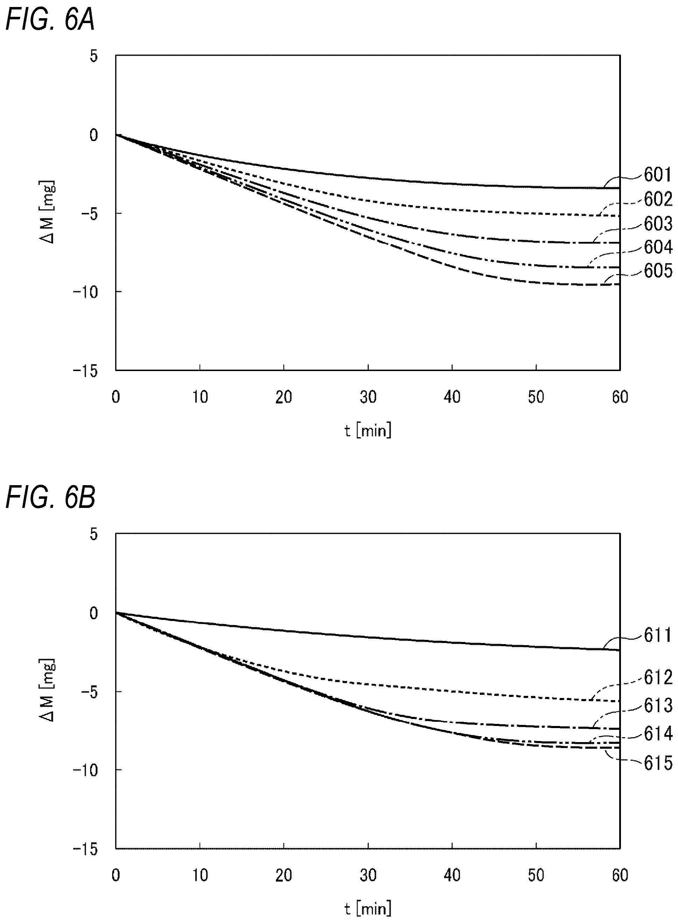

Here, the influence of the parameters used in the calculation formula for calculating the evaporation of the grease will be described. FIGS. 6A to 6E are graphs illustrating the influence of the parameters on the evaporation amount. In FIGS. 6A to 6E, a horizontal axis represents the time t [min], and a vertical axis represents the change amount $\Delta M$ [mg].

In addition, the calculation conditions at the time of measuring the parameters are as follows, and all are common.

> Initial amount of grease: 10 [mg]
> Base oil: PAO2
> Temperature: 180 [°C]
> Diameter of container: 5 [mm]
> Height of container: 5 [mm]

FIG. 6A is a graph illustrating a change in the change amount $\Delta M$ when a concentration $1-\varepsilon$ of a thickener is changed. Solid lines 601, 602, 603, 604, and 605 in this order indicate cases where the density is 0.60, 0.45, 0.30, 0.15, and 0.05, respectively. According to the result in FIG. 6A, the evaporation of the grease (that is, the base oil) is more restricted as the concentration of the thickener is higher.

FIG. 6B is a graph illustrating a change in the change amount $\Delta M$ when the fitting parameter $\delta$ is changed. Solid lines 611, 612, 613, 614, and 615 in this order indicate cases where $\delta$ is $1.5 \times 10^{-30}$, $1.5 \times 10^{-28}$, $1.5 \times 10^{-26}$, $1.5 \times 10^{-24}$, and $1.5 \times 10^{-22}$, respectively. According to the result in FIG. 6B, the more the evaporation of the grease (that is, the base oil) is more restricted as $\delta$ is smaller. A small $\delta$ corresponds to a large specific area of the thickener. In other words, this corresponds to the presence of more irregularities due to a surface of the thickener.

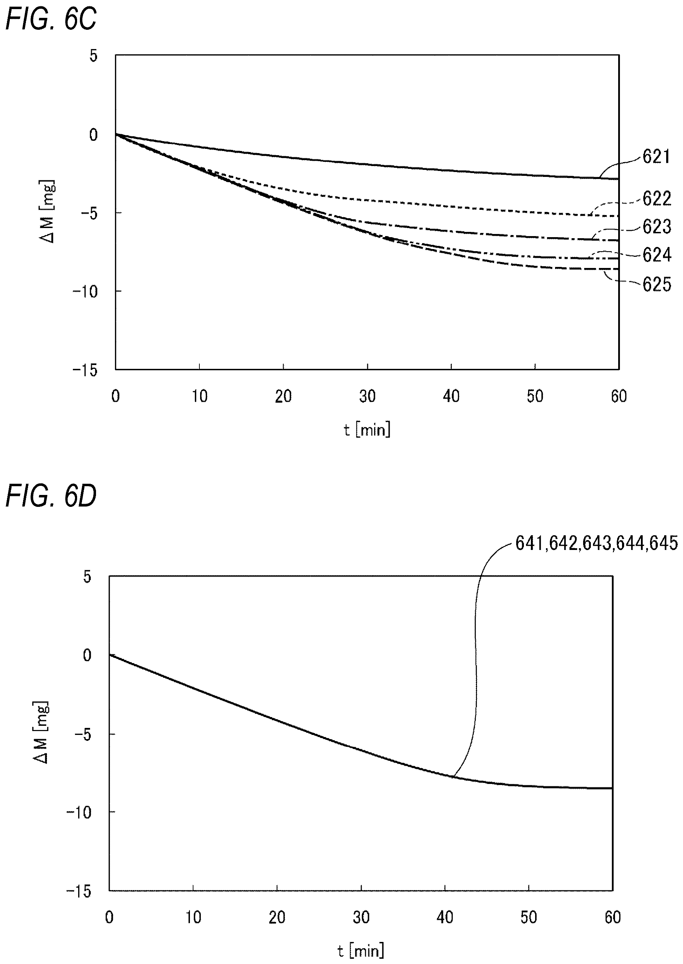

FIG. 6C is a graph illustrating a change in the change amount $\Delta M$ when the fitting parameter k is changed. Solid lines 621, 622, 623, 624, and 625 in this order indicate cases where k is 5400, 1800, 600, 150, and 15, respectively. According to the result in FIG. 6C, the evaporation of the grease (that is, the base oil) is more restricted as k is larger. A large k corresponds to a long true flow path in the grease. In other words, this corresponds to a structure in which the mesh of the thickener is more intricately entangled.

FIG. 6D is a graph illustrating a change in the change amount $\Delta M$ when the fitting parameter m is changed. Here, m was changed to 0.001, 0.01, 0.1, 8, and 80, but there was no influence on the value as indicated by solid lines 641 to 645. According to the result in FIG. 6D, the value of m does not affect the evaporation of the grease (that is, the base oil).

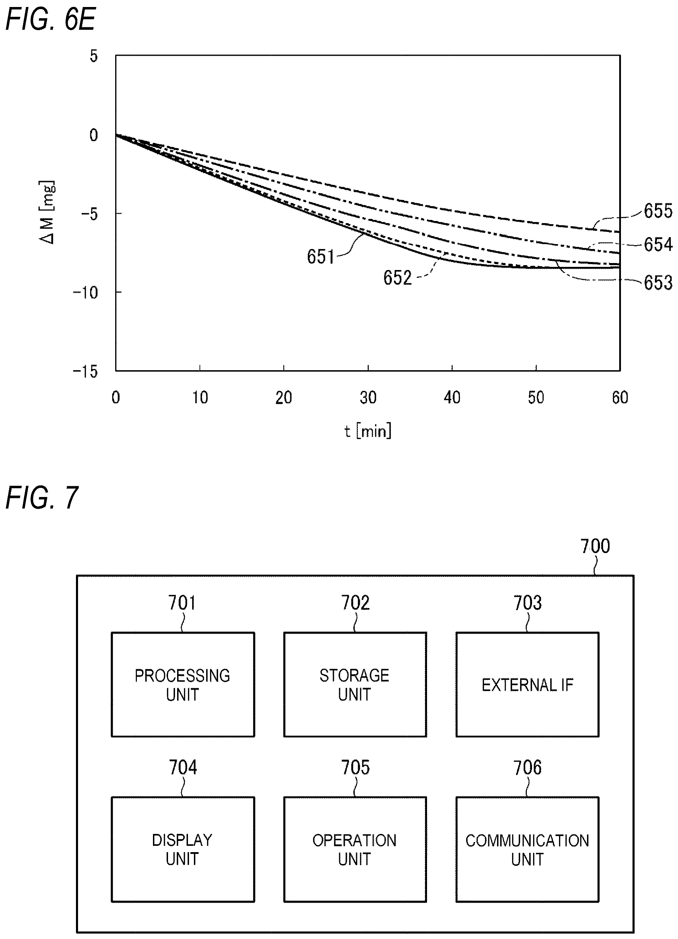

FIG. 6E is a graph illustrating a change in the change amount $\Delta M$ when the molecular weight $M_{thick}$ of the thickener is changed. Solid lines 651, 652, 653, 654, and 655 in this order indicate cases of 1600, 800, 320, 160, and 80, respectively. According to the result in FIG. 6E, the evaporation of the grease (that is, the base oil) is more restricted as $M_{thick}$ is larger.

[Device Configuration]

FIG. 7 is a diagram illustrating a configuration example of an information processing device capable of measuring or predicting evaporation of grease using the method according to the present embodiment. An information processing device 700 includes a processing unit 701, a storage unit 702, an external interface (IF) 703, a display unit 704, an operation unit 705, and a communication unit 706. The information processing device 700 may be implemented as, for example, a general-purpose information processing device such as a personal computer (PC) or may be implemented as a dedicated device.

The processing unit 701 may include a central processing unit (CPU), a micro processing unit (MPU), a digital single processor (DSP), or a dedicated circuit. The storage unit 702 includes volatile and nonvolatile storage media such as a hard disk drive (HDD), a read only memory (ROM), and a random access memory (RAM), and may input and output various types of information in response to an instruction from the processing unit 701. The processing unit 701 may implement the above-described calculation method by reading and executing a program, an application, and various types of data related to the calculation method according to the present embodiment from the storage unit 702.

The external IF 703 is an interface for connecting to an external device. For example, when the method according to the present embodiment is used at the time of measurement, the external IF 703 may be connected to a sensor or the like for measuring information such as a temperature or an atmospheric pressure in a surrounding environment of a measurement object. The display unit 704 is a part for outputting a result obtained by the method according to the present embodiment, and performs output to a user according to an instruction from the processing unit 701. The operation unit 705 is a part for receiving input of parameters and input of various instructions from the user. The communication unit 706 is a network interface for communicating with an external device, and may be configured to output a result obtained by the method according to the present embodiment to the outside, for example.

In the information processing device as described above, it is possible to predict the evaporation amount of the grease with the lapse of time by setting the fitting parameters, which are obtained by the above-described equations and fitting, and receiving the input of the elapsed time ($\Delta t$) from the initial time and the initial grease height (z) from the measurement object.

As described above, according to the configuration of the present embodiment, it is possible to easily predict the evaporation amount of the grease. In particular, the evaporation amount can be predicted in consideration of the influence of the vapor pressure depression and the capillary force according to the configuration of the grease.

<Other Embodiments>

In the present invention, programs or applications for implementing the functions of the one or more embodiments described above may be supplied to a system or a device using a network or a storage medium, and the functions may also be implemented in processing in which one or more processors in a computer of the system or the device read and execute the programs.

In addition, the functions may be implemented by a circuit (for example, an application specific integrated circuit (ASIC) or a field programmable gate array (FPGA)) that implements one or more functions.

As described above, the present invention is not limited to the embodiments described above, and combinations of the respective configurations of the embodiments and modifications and applications by those skilled in the art based on the descriptions in the description and the well-known technique are intended by the present invention and are thus also included within the scope of the present invention to be protected.

As described above, the following matters are disclosed in the present specification.

(1) A prediction method for predicting an evaporation amount of grease, the prediction method including:

a calculation step of calculating a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and
an output step of outputting the calculated change amount as an evaporation amount of the grease.

According to this configuration, it is possible to easily predict the evaporation amount of the grease. In particular, the evaporation amount can be predicted in consideration of the influence of the vapor pressure depression and the capillary force according to the configuration of the grease.

(2) The prediction method according to (1), in which the first equation is defined by a following equation.

[Math. 10]

$$N_m = \frac{D_{AB} P'_{vap}}{y_{B,lm} RTZ}$$

$$D_{AB} = (1.858 \times 10^{-4}) \frac{\left(\frac{1}{M_A} + \frac{1}{M_B}\right)^{\frac{1}{2}} T^{\frac{3}{2}}}{P \sigma_{AB}^2 \Omega_D}$$

$$P'_{vap} = \frac{n_{oil}}{n_{oil} + n_{thick}} P_{vap}$$

$$y_{B,lm} = \frac{y_{B2} - y_{B1}}{\ln\left(\frac{y_{B2}}{y_{B1}}\right)}$$

$N_m$: first molar mass [mol/s·m$^2$]
P: absolute pressure [Pa]
$P_{vap}$: vapor pressure of base oil [Pa]
$n_{oil}$: molar mass of base oil [mol]
$n_{thiek}$: molar mass of thickener [mol]
$D_{AB}$: two-component (vaporized base oil and atmosphere gas) diffusion coefficient [m$^2$/s]
$y_B$, lm: values that changes due to influence of convection in atmosphere gas [-]
R: gas constant [J/K·mol]
T: temperature [K]

Z: distance from top surface of container to liquid surface (= $z_2$ - $z_1$) [m]
$z_1$: liquid surface height [m]
$z_2$: container height (> $z_1$) [m]
$y_{B1}$: molar fraction of gas B at position of liquid surface height $z_1$ [-]
$y_{B2}$: molar fraction of gas B at container height $z_2$ [-]
$M_A$: molecular weight of vaporized base oil [g/mol]
$M_B$: molecular weight of atmosphere gas [g/mol]
$\sigma_{AB}$: collision distance (= ($\sigma_A$ + $\sigma_B$)/2) [nm]
$\sigma_A$: molecular diameter of vaporized base oil [nm]
$\sigma_B$: molecular diameter of atmosphere gas [nm]
$\Omega_D$: collision integral [-]

According to this configuration, it is possible to predict the evaporation amount in consideration of the influence of the vapor pressure depression according to the configuration of the grease.

(3) The prediction method according to (1) or (2), in which the second equation is defined by a following equation.

[Math. 11]

$$N_c = \frac{1}{2}\sqrt{\frac{\delta\varepsilon^2}{k'k^3(1-\varepsilon)^{2+m}}\frac{\rho T\cos\theta}{M\mu z}}$$

$$k = 2k'\left(\frac{z_a}{z}\right)$$

$N_c$: second molar mass [mol/s·m$^2$]
$\delta$: fitting parameter [m$^2$]
$\varepsilon$: particle layer void ratio [-]
k: fitting parameter [-]
k': fitting parameter [-]
m: fitting parameter [-]
$\rho$: density of base oil [g/m$^3$]
T: temperature [K]
$\theta$: contact angle of base oil to particle [rad.]
M: molecular weight [g/mol]
$\mu$: viscosity of base oil [Pa·s]
z: length of particle layer [m]
$z_a$: average length of curved flow path [m]

According to this configuration, it is possible to predict the evaporation amount in consideration of the influence of the capillary force according to the configuration of the grease.

The prediction method according to any one of (1) to (3), in which in the calculation step, the change amount of the grease is calculated using a third equation defined by a following equation.

[Math. 12]

$$z_{t+\Delta t} = z_t + \Delta z = z_t - \frac{M}{\rho}N\Delta t$$

$$N = Min(N_m, N_c)$$

$z_t$: liquid surface height at time point t [m]

$z_{t+\Delta t}$: liquid surface height after elapse of time t from time point t [m]

$\Delta z$: change amount of z [m]

M: molecular weight [g/mol]

$\rho$: density of liquid [g/m$^3$]

N: molar mass by which liquid moves per unit time and per unit area [mol/s·m$^2$]

$\Delta t$: elapsed time [s]

$N_m$: first molar mass [mol/s·m$^2$]

$N_c$: second molar mass [mol/s·m$^2$]

According to this configuration, it is possible to predict the evaporation amount in consideration of the influence of the vapor pressure depression and the capillary force according to the configuration of the grease.

(5) A prediction device for predicting an evaporation amount of grease, the prediction device including:

a calculation unit configured to calculate a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output unit configured to output the calculated change amount as an evaporation amount of the grease.

According to this configuration, it is possible to easily predict the evaporation amount of the grease. In particular, it is possible to predict the evaporation amount in consideration of the influence of the vapor pressure depression and the capillary force according to the configuration of the grease.

(6) A program for causing a computer to execute:

a calculation step of calculating a change amount of grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output step of outputting the calculated change amount as an evaporation amount of the grease.

According to this configuration, it is possible to easily predict the evaporation amount of the grease. In particular, it is possible to predict the evaporation amount in consideration of the influence of the vapor pressure depression and the capillary force according to the configuration of the grease.

Although various embodiments have been described above with reference to the drawings, it is needless to mention that the present invention is not limited to these examples. It is apparent to those skilled in the art that various modifications or corrections can be conceived within the scope described in the claims, and it is understood that the modifications or corrections naturally fall within the technical scope of the present invention. In addition, the components described in the above embodiments may be combined in any manner without departing from the gist of the invention.

The present application is based on a Japanese patent application (No. 2023-088048) filed on May 29, 2023, contents of which are incorporated herein by reference.

INDUSTRIAL APPLICABILITY

The present invention has an effect that the evaporation amount of grease can be easily predicted, and can be used for, for example, a rolling bearing, a rotation device, and a sliding device, but is not limited thereto, and can be used in an environment where grease is used or designed.

REFERENCE SIGNS LIST

700: information processing device
701: processing unit
702: storage unit
703: external IF
704: display unit
705: operation unit
706: communication unit

**Claims**

1. A prediction method for predicting an evaporation amount of grease, the prediction method comprising:

   a calculation step of calculating a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and
   an output step of outputting the calculated change amount as an evaporation amount of the grease.

2. The prediction method according to claim 1, wherein

   the first equation is defined by a following equation,

   [Math. 1]

   $$N_m = \frac{D_{AB} P'_{vap}}{y_{B,lm} R T Z}$$

   $$D_{AB} = (1.858 \times 10^{-4}) \frac{\left(\frac{1}{M_A} + \frac{1}{M_B}\right)^{\frac{1}{2}} T^{\frac{3}{2}}}{P \sigma_{AB}{}^2 \Omega_D}$$

   $$P'_{vap} = \frac{n_{oil}}{n_{oil} + n_{thick}} P_{vap}$$

   $$y_{B,lm} = \frac{y_{B2} - y_{B1}}{\ln\left(\frac{y_{B2}}{y_{B1}}\right)}$$

   in which $N_m$: first molar mass [mol/s·m$^2$],
   P: absolute pressure [Pa],
   $P_{vap}$: vapor pressure of base oil [Pa],
   $n_{oil}$: molar mass of base oil [mol],
   $n_{thick}$: molar mass of thickener [mol],
   $D_{AB}$: two-component (vaporized base oil and atmosphere gas) diffusion coefficient [m$^2$/s],
   $y_B$, lm: values that change due to influence of convection in atmosphere gas [-],
   R: gas constant [J/K·mol],
   T: temperature [K],
   Z: distance from top surface of container to liquid surface (= $z_2$ - $z_1$) [m],
   $z_1$: liquid surface height [m],
   $z_2$: container height (> $z_1$) [m],
   $y_{B1}$: molar fraction of gas B at position of liquid surface height $z_1$ [-],
   $y_{B2}$: molar fraction of gas B at container height $z_2$ [-],
   $M_A$: molecular weight of vaporized base oil [g/mol],
   $M_B$: molecular weight of atmosphere gas [g/mol],
   $\sigma_{AB}$: collision distance (= ($\sigma_A$ + $\sigma_B$)/2) [nm],
   $\sigma_A$: molecular diameter of vaporized base oil [nm],
   $\sigma_B$: molecular diameter of atmosphere gas [nm], and $\Omega_D$: collision integral [-].

3. The prediction method according to claim 1, wherein

the second equation is defined by a following equation,

[Math. 2]

$$N_c = \frac{1}{2} \sqrt{\frac{\delta \varepsilon^2}{k' k^3 (1 - \varepsilon)^{2+m}} \frac{\rho T \cos \theta}{M \mu z}}$$

$$k = 2k' \left( \frac{z_a}{z} \right)$$

in which $N_c$: second molar mass [mol/s·m$^2$],
$\delta$: fitting parameter [m$^2$],
$\varepsilon$: particle layer void ratio [-],
k: fitting parameter [-],
k': fitting parameter [-],
m: fitting parameter [-],
$\rho$: density of base oil [g/m$^3$],
T: temperature [K],
$\theta$: contact angle of base oil to particle [rad.],
M: molecular weight [g/mol],
$\mu$: viscosity of base oil [Pa·s],
z: length of particle layer [m], and
$z_a$: average length of curved flow path [m].

4. The prediction method according to any one of claims 1 to 3, wherein

in the calculation step, the change amount of the grease is calculated using a third equation defined by a following equation,

[Math. 3]

$$z_{t+\Delta t} = z_t + \Delta z = z_t - \frac{M}{\rho} N \Delta t$$

$$N = \text{Min}(N_m, N_c)$$

in which $z_t$: liquid surface height at time point t [m],
$z_{t+\Delta t}$: liquid surface height after elapse of time t from time point t [m],
$\Delta z$: change amount of z [m],
M: molecular weight [g/mol],
$\rho$: density of liquid [g/m$^3$],
N: molar mass by which liquid moves per unit time and per unit area [mol/s·m$^2$],
$\Delta t$: elapsed time [s],
$N_m$: first molar mass [mol/s·m$^2$], and
$N_c$: second molar mass [mol/s·m$^2$].

5. A prediction device for predicting an evaporation amount of grease, the prediction device comprising:

a calculation unit configured to calculate a change amount of the grease using a value of a smaller molar mass among a first molar mass obtained by a first equation based on a vapor pressure depression due to a base oil and a thickener contained in the grease and a second molar mass obtained by a second equation based on a capillary force due to the thickener; and

an output unit configured to output the calculated change amount as an evaporation amount of the grease.

6. A program for causing a computer to execute:

a calculation step of calculating a change amount of grease using a value of a smaller molar mass among a first molar mass obtained by a first equation based on a vapor pressure depression due to a base oil and a thickener contained in the grease and a second molar mass obtained by a second equation based on a capillary force due to the thickener; and
an output step of outputting the calculated change amount as an evaporation amount of the grease.

*FIG. 1*

EP 4 722 690 A1

*FIG. 2*

## FIG. 3

*FIG. 4*

FIG. 5

FIG. 6A

FIG. 6B

*FIG. 6C*

*FIG. 6D*

*FIG. 6E*

*FIG. 7*

## INTERNATIONAL SEARCH REPORT

| | International application No. |
|---|---|
| | **PCT/JP2024/019681** |

**A. CLASSIFICATION OF SUBJECT MATTER**

*G01N 7/00*(2006.01)i; *C10M 169/02*(2006.01)i; *C10N 40/00*(2006.01)n; *C10N 50/10*(2006.01)n
FI: G01N7/00 Z; C10M169/02; C10N50:10; C10N40:00 Z

According to International Patent Classification (IPC) or to both national classification and IPC

**B. FIELDS SEARCHED**

Minimum documentation searched (classification system followed by classification symbols)

G01N7/00; C10M169/02; C10N40/00; C10N50/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2013-116991 A (NSK LTD.) 13 June 2013 (2013-06-13)<br>entire text, all drawings | 1-6 |
| A | JP 2004-26941 A (NTN CORP.) 29 January 2004 (2004-01-29)<br>entire text, all drawings | 1-6 |
| A | JP 2020-165878 A (HITACHI, LTD.) 08 October 2020 (2020-10-08)<br>entire text, all drawings | 1-6 |
| A | JP 2021-63183 A (KYODO YUSHI CO., LTD.) 22 April 2021 (2021-04-22)<br>entire text, all drawings | 1-6 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
**Information on patent family members**

International application No.

**PCT/JP2024/019681**

| Patent document cited in search report | | | Publication date (day/month/year) | Patent family member(s) | | Publication date (day/month/year) |
|---|---|---|---|---|---|---|
| JP | 2013-116991 | A | 13 June 2013 | (Family: none) | | |
| JP | 2004-26941 | A | 29 January 2004 | (Family: none) | | |
| JP | 2020-165878 | A | 08 October 2020 | (Family: none) | | |
| JP | 2021-63183 | A | 22 April 2021 | WO | 2021/075553 A1 | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5319995 B

- JP 2023088048 A

**Non-patent literature cited in the description**

- **SOMA MINORU et al.** Measurement of grease permeability using centrifugal oil separation tests. *Tribology Conference 2018 Autumn Proceedings*

- **SOMA MINORU et al.** Measurement of grease permeability using centrifugal oil separation tests (Second report). *Tribology Conference 2019 Spring Proceedings*